# EUROPEAN PATENT APPLICATION

(11) **EP 2 842 941 A1**
(43) Date of publication of application: **04.03.2015**
(21) Application number: 13782308.4
(22) Date of filing: 06.03.2013
(51) Int. Cl.: C07C 381/00

(54) **METHOD FOR PRODUCING TRIMETHYLSULFOXONIUM BROMIDE AND TEMPERATURE CONTROL METHOD FOR REACTION SYSTEM PRODUCING TRIMETHYLSULFOXONIUM BROMIDE**

(30) Priority: 24.04.2012 JP 2012099132
(71) Applicant: Kureha Corporation, Chuo-ku Tokyo 103-8552 (JP)
(72) Inventor: OOHASHI, Takashi, Tokyo 103-8552 (JP); ARAKI, Nobuyuki, Tokyo 103-8552 (JP)
(74) Representative: Hoffmann Eitle
(86) International application number: PCT/JP2013/056106
(87) International publication number: WO 2013/161394

(57) **Abstract**

The present invention relates to a method of producing trimethylsulfoxonium bromide by reacting dimethyl sulfoxide with methyl bromide (MeBr), wherein MeBr is added in a manner that satisfies conditions (1) and (2): (1) MeBr is added at an addition rate that complies with a predetermined feeding profile, (2) MeBr is added upon the temperature in the reaction system reaching a predetermined maximum temperature.

## Description

### TECHNICAL FIELD

The present invention relates to a method of producing trimethylsulfoxonium bromide by reacting dimethyl sulfoxide with methyl bromide.

### BACKGROUND ART

Trimethylsulfoxonium bromide is a compound that is useful as an intermediate of antimicrobial azole derivatives. Trimethylsulfoxonium bromide is produced by reacting dimethyl sulfoxide with methyl bromide. A method of producing trimethylsulfoxonium bromide by reacting dimethyl sulfoxide with methyl bromide has been disclosed in Patent Document 1, for example, which describes a method of producing trimethylsulfoxonium bromide by adding methyl bromide in slight increments to dimethyl sulfoxide heated to a temperature of from 50 to 75°C at atmospheric pressure, followed ultimately by reacting from 0.40 to 0.70 mols of methyl bromide per mol of dimethyl sulfoxide.

### CITATION LIST

### Patent Literature

Patent Document 1: Japanese Unexamined Patent Application Publication No. H9-235268A (publication date September 9, 1997).

### SUMMARY OF INVENTION

### Technical Problem

Dimethyl sulfoxide, the starting material in the production of trimethylsulfoxonium bromide, undergoes a decomposition reaction as the reaction temperature rises and as acid becomes admixed in the reaction solution. When a decomposition reaction starts, acid is produced and that acid then further promotes the decomposition reaction, which vastly stimulates the decomposition reaction in one stroke. Furthermore, even in the case of the reaction solution being set aside at thermal insulation, the temperature of the reaction solution rises due to autolytic heat storage and the decomposition reaction is accelerated.

Since decomposition cannot be halted once a decomposition reaction starts, there is a danger of an explosion occurring because of the great stimulation of the decomposition reaction. Accordingly, appropriate control of the reaction temperature of dimethyl sulfoxide and methyl bromide in the production of trimethylsulfoxonium bromide is crucial.

The present invention was devised in light of the aforementioned problems. An object is to provide a method of producing trimethylsulfoxonium bromide in which the reaction temperature of dimethyl sulfoxide and methyl bromide is controlled appropriately and easily.

### Solution To Problem

In order to resolve the aforementioned issues, the present invention provides a method of producing trimethylsulfoxonium bromide by reacting dimethyl sulfoxide with methyl bromide in which
methyl bromide is added to dimethyl sulfoxide in a manner that satisfies (1) and (2) below:
(1) methyl bromide is added at an addition rate that complies with a predetermined feeding profile based on the reaction rate estimated from the reaction temperature,
(2) the temperature in the reaction system of dimethyl sulfoxide and methyl bromide is detected and methyl bromide is added upon the detected temperature reaching a predetermined maximum temperature.

The present invention, in order to resolve the aforementioned issues, also provides a method of producing trimethylsulfoxonium bromide by reacting dimethyl sulfoxide with methyl bromide in which
methyl bromide is added to dimethyl sulfoxide in a manner that satisfies (1) and (2) below:
(1) the temperature in the reaction system is detected and methyl bromide is added upon the detected temperature reaching a predetermined maximum temperature,
(2) the addition rate of methyl bromide is reduced or addition is suspended upon the aforementioned detected temperature falling below a predetermined minimum temperature.

The present invention, in order to resolve the aforementioned issues, further provides a method of controlling the temperature of the reaction system that produces trimethylsulfoxonium bromide by reacting dimethyl sulfoxide with methyl bromide in which the temperature of the aforementioned reaction system is detected, methyl bromide is added upon the detected temperature reaching a predetermined maximum temperature, and the addition rate of methyl bromide is reduced or addition is suspended upon the temperature falling below a predetermined minimum temperature.

### EFFECT OF THE INVENTION

In the method of producing trimethylsulfoxonium bromide according to the present invention, methyl bromide is added to dimethyl sulfoxide in a manner that satisfies conditions (1) and (2) below: (1) methyl bromide is added at an addition rate that complies with a predetermined feeding profile based on the reaction rate estimated from the target reaction temperature, (2) the temperature in the reaction system of dimethyl sulfoxide and methyl bromide is detected and methyl bromide is added upon the detected temperature reaching a predetermined maximum temperature. The temperature of the reaction system can be controlled and the control can be completed without reliance on external modalities such as a jacket because the temperature can be controlled by adding methyl bromide. Accordingly, the reaction can be implemented easily, efficiently and stably while inhibiting temperature elevation in the reaction system.

### BRIEF DESCRIPTION OF THE DRAWING

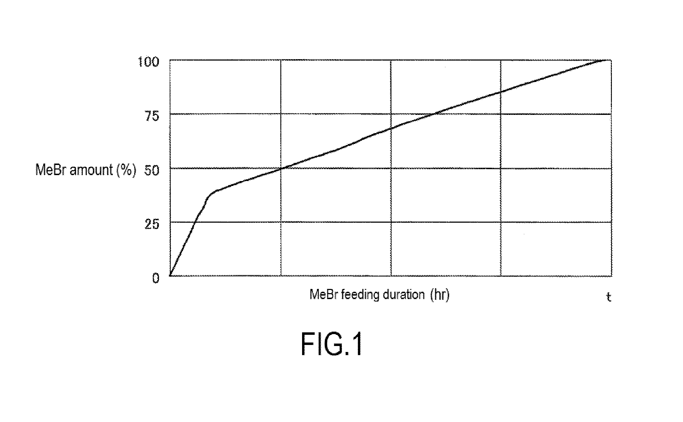

FIG. 1 illustrates an example of a methyl bromide feeding profile.

### DETAILED DESCRIPTION

One embodiment of the method of producing trimethylsulfoxonium bromide according to the present invention is explained below.

The method of producing trimethylsulfoxonium bromide in the present embodiment is a method of producing trimethylsulfoxonium bromide by reacting dimethyl sulfoxide with methyl bromide in which methyl bromide is added to dimethyl sulfoxide in a manner that satisfies (1) and (2) below:
(1) methyl bromide is added at an addition rate that complies with a predetermined feeding profile based on the reaction rate estimated from the target reaction temperature.
(2) the temperature in the reaction system of dimethyl sulfoxide and methyl bromide is detected and methyl bromide is added upon the detected temperature reaching a predetermined maximum temperature.

The feeding profile represents the relation between the duration of time from the start of the reaction and the total amount of methyl bromide added up to that point, both being set based on the reaction rate estimated from the target reaction temperature. The term "target reaction temperature" refers to the preset target temperature in the reaction system. It does not refer to the actual detected temperature in the reaction system. Moderate reflux of methyl bromide can be maintained by following the feeding profile, and the temperature in the reaction system can be stably controlled. An example of a feeding profile is illustrated in FIG. 1. Time advances from the left to the right in the profile illustrated in FIG. 1. In addition, the ordinate represents the total amount of methyl bromide added at each time period when the total amount of methyl bromide that is ultimately added is taken as 100%. The reaction rate, specifically, the rate of methyl bromide consumption, is dependent on the temperature of the reaction system and on the concentration of the starting material. Accordingly, the general consumption rate of methyl bromide is determined upon the target reaction temperature being determined. In the method of producing trimethylsulfoxonium bromide in the present embodiment, as described below, heat is removed from the reaction system by utilizing the latent heat of evaporation of methyl bromide. Consequently, in the reaction system, the addition of methyl bromide is preferably commensurate with the consumption rate of methyl bromide in order to prevent excessive cooling in the reaction system and a decline in the reaction rate and also to prevent temperature elevation in the reaction system due to a methyl bromide insufficiency. Furthermore, stable heat removal through moderate methyl bromide reflux is preferable. The feeding profile is predetermined from such a perspective.

The feeding profile is an index for carrying out stable heat removal while maintaining moderate reflux. The aforementioned (1) feeding conditions in the method of producing trimethylsulfoxonium bromide in the present embodiment are not intended to limit the total amount of methyl bromide addition at a given moment in time so as to completely match the predetermined feeding profile.

Furthermore, in the method of producing trimethylsulfoxonium bromide in the present embodiment, the temperature in the reaction system of dimethyl sulfoxide and methyl bromide is detected, and methyl bromide is added upon the detected temperature reaching the predetermined maximum temperature.

In the method of producing trimethylsulfoxonium bromide in the present embodiment, heat is removed from the reaction system using of the latent heat of evaporation of methyl bromide. Specifically, evaporated methyl bromide is condensed in a condenser and heat from the reaction system is removed by evaporating the condensed methyl bromide in the reaction system. The latent heat of evaporation increases because the amount of evaporation of methyl bromide increases with the addition of methyl bromide, resulting in a decline of the temperature of the reaction system. Specifically, when the temperature in the reaction system reaches a predetermined maximum temperature, the temperature of the reaction system is lowered by adding methyl bromide to the reaction system, which enables the temperature to be kept in control at a temperature that is lower than the maximum temperature. Accordingly, the decomposition of dimethyl sulfoxide due to temperature elevation in the reaction system can be prevented by adding methyl bromide when the temperature in the reaction system has risen.

Setting the maximum temperature to a temperature that is higher than the target reaction temperature but that is equal to or less than 20°C above the target reaction temperature is preferable from the perspective of preventing a decomposition reaction from occurring. Setting the maximum temperature to a temperature that is equal to or less than 5.0°C above the target reaction temperature is more preferable.

Furthermore, in the method of producing trimethylsulfoxonium bromide of the present embodiment, the temperature in the reaction system of dimethyl sulfoxide and methyl bromide is detected, and the addition rate of methyl bromide is reduced or methyl bromide addition is suspended if the detected temperature falls below the predetermined minimum temperature. The amount of methyl bromide evaporation decreases if the addition rate of methyl bromide is reduced or if its addition is suspended, and the latent heat of evaporation also decreases. As a result, the temperature in the reaction system rises. Specifically, the temperature of the reaction system is elevated, which enables the temperature in the reaction system to be controlled at a temperature higher than the minimum temperature, by reducing the addition rate or by suspending the addition of methyl bromide when the temperature in the reaction system falls below the predetermined minimum temperature. Accordingly, prolongation of the time required until completion of the reaction due to a decline in the temperature of the reaction system can be prevented by reducing the addition rate of methyl bromide or by suspending the addition of methyl bromide when the temperature in the reaction system has fallen.

Setting the minimum temperature to one that is lower than the target reaction temperature but that is equal to or higher than 10°C below the target reaction temperature is preferable from the perspective of contracting the duration of time until completion of the reaction. Setting the minimum temperature to one that is equal to or higher than 5.0°C below the target reaction temperature is more preferable.

In the method of producing trimethylsulfoxonium bromide of the present embodiment, methyl bromide is added when the detected temperature reaches the maximum temperature while the addition rate of methyl bromide is reduced or methyl bromide addition is suspended when the detected temperature falls below the minimum temperature. Methyl bromide addition is permissible or the stoppage of its addition is also permissible if the detected temperature is equal to or higher than the minimum temperature and below the maximum temperature. The decision concerning adding or not adding methyl bromide when the temperature in the reaction system is in this range may be determined by whether the temperature in the reaction system at that moment is tending to rise or tending to fall. In other words, the addition of methyl bromide may be determined based on whether or not moderate reflux of methyl bromide can be maintained.

In the method of producing trimethylsulfoxonium bromide of the present embodiment, detection of the temperature in the reaction system may be constantly carried out or at fixed intervals. When the temperature is measured constantly, the addition rate may be altered over time in response to changes in the detected temperature, for example. When the temperature is measured at fixed intervals, for example, from a given measurement until the next measurement, the addition rate may be maintained at a constant based on the temperature detected at the given measurement.

As indicated above, the temperature in the reaction system is detected and decomposition of dimethyl sulfoxide can be prevented by preventing temperature elevation of the reaction system through the addition of methyl bromide when the detected temperature has reached a predetermined maximum temperature. In addition, a decline in the reaction rate can be prevented by preventing a decline in the temperature of the reaction system by decreasing the addition rate of methyl bromide or by suspending its addition when the detected temperature falls below the predetermined minimum temperature. Accordingly, in the reaction that produces trimethylsulfoxonium bromide by reacting dimethyl sulfoxide with methyl bromide, the temperature in the reaction system is detected and methyl bromide is added when the detected temperature reaches a predetermined maximum temperature while the addition rate of methyl bromide is reduced or methyl bromide addition is suspended when the detected temperature falls below a predetermined minimum temperature. This method of controlling the temperature of the reaction system is included in the scope of the present invention.

In the method of producing trimethylsulfoxonium bromide in the present embodiment, while the reaction of dimethyl sulfoxide and methyl bromide is carried out, the reaction vessel where the reaction takes place is heated using a temperature regulating apparatus such as a jacket, oil bath, or hot water bath. The amount of methyl bromide evaporation is increased by heating the reaction vessel and the amount of reflux is also increased, as a result of which the amount of heat removal can be increased. Heating the reaction vessel so that its temperature can be maintained at from 0°C to 20°C above the target reaction temperature is preferable. Heating the reaction vessel so that its temperature can be maintained at from 0°C to 10°C above the target reaction temperature is more preferable. In an easier method of temperature adjustment, more preferably, the temperature of a temperature regulating apparatus such as a jacket is held constant and adjusting the addition rate of methyl bromide alone controls the temperature in the reaction system. In this specification, "maintained at a temperature 0°C above the target reaction temperature" refers to maintenance at the target reaction temperature.

In the present embodiment, removal of heat from the reaction system (cooling) is carried out only by evaporation, condensation, and reflux of methyl bromide. Furthermore, a temperature regulating apparatus such as a jacket is usually used during a reaction to heat the reaction vessel, and not used as an external means of cooling. However, since the temperature regulating apparatus such as a jacket can be used as an external cooling means, if the temperature of the reaction system rises unexpectedly such that emergency reduction of the reaction system temperature becomes necessary, such rapid temperature elevation can be handled expeditiously without provision of another cooling means by switching the temperature regulating apparatus such as a jacket that had been used for heating to an external cooling means.

In the method of producing trimethylsulfoxonium bromide in the present embodiment, the amount of methyl bromide per mol of dimethyl sulfoxide that is used is, for example, from 0.40 to 0.70 mol, preferably from 0.45 to 0.55 mol, and more preferably from 0.48 to 0.53 mols. If the amount of methyl bromide use is equal to or greater than 0.40 mols, the amount of product dissolved in the filtrate when isolating the product can be reduced to a relatively low level, and the isolation yield can be raised. Furthermore, the time required for the reaction can be contracted by setting the amount of methyl bromide in use at equal to or less than 0.70 mols.

In the method of producing trimethylsulfoxonium bromide of the present embodiment, at least one compound selected from the group consisting of trimethyl orthoformate, triethyl orthoformate, tripropyl orthoformate, tetramethyl orthocarbonate, tetraethyl orthocarbonate, tetraisopropyl orthocarbonate, and tetrapropyl orthocarbonate (hereinafter referred to as trimethyl orthoformate and the like) is preferably contained in the reaction system. These compounds are surmised to function as auxiliary agents in the acquisition of substances that promote dimethyl sulfoxide decomposition. Accordingly, the decomposition of dimethyl sulfoxide can be more reliably inhibited by ensuring the presence of the aforementioned compound(s) in the reaction system.

The amount of trimethyl orthoformate and the like used is, for example, from 0.002 to 0.05 mols per mol of dimethyl sulfoxide, and preferably from 0.004 to 0.02 mols.

The moisture concentration in the reaction system is preferably not greater than 400 ppm, more preferably not greater than 200 ppm, and even more preferably not greater than 100 ppm when trimethyl orthoformate and the like is contained in the reaction system. Trimethyl orthoformate and the like is a compound that decomposes upon reaction with water. Consequently, the decomposition of trimethyl orthoformate and the like can be inhibited by setting the moisture concentration in the reaction system at not greater than 400 ppm. Methods of setting the moisture concentration in the reaction system at not greater than 400 ppm include for example, 1) a drying method in which the starting material compounds dimethyl sulfoxide, methyl bromide, and trimethyl orthoformate and the like are dried using a desiccant such as a molecular sieve, 2) a drying method in which low boiling-point compounds containing trimethyl orthoformate are distilled off to the extent possible following the addition of trimethyl orthoformate to dimethyl sulfoxide and heating, or 3) a drying method in which the starting material compound dimethyl sulfoxide is subjected to heating reflux in the presence of CaH, followed by distillation under vacuum.

The method of producing trimethylsulfoxonium bromide in the present embodiment can be run either at atmospheric pressure or at micropressure. In this specification, "micropressure" refers to pressure of not greater than approximately 10 kPa. So long as methyl bromide is refluxed at the target reaction temperature, both micropressure and atmospheric pressure are permissible, but micropressure is preferable. The admixture of moisture from outside of the reaction system into the reaction system can be prevented by setting the system at micropressure.

In the method of producing trimethylsulfoxonium bromide in the present embodiment, stirring at the same temperature as the temperature at addition is carried out for 3 to 24 hours after the addition of a predetermined amount of methyl bromide. Following completion of the reaction, isolation and refinement may be carried out using conventional refining techniques. For example, the reaction mixture may be cooled to room temperature, followed by filtration of the insoluble portion. The insoluble portion (filtered material) separated by filtration is washed using an organic solvent such as benzene, toluene and xylene. This filtered material is dried in a nitrogen stream or under vacuum at a temperature of from 30 to 60°C to complete isolation of trimethylsulfoxonium bromide.

As described above, in the method of producing trimethylsulfoxonium bromide according to the present invention, the aforementioned maximum temperature is preferably higher than the aforementioned target reaction temperature and not higher than 5.0°C above the aforementioned target reaction temperature.

Furthermore, in the method of producing trimethylsulfoxonium bromide according to the present invention, the addition rate of methyl bromide is reduced or methyl bromide addition is suspended if the aforementioned detected temperature falls below the predetermined minimum temperature

In the method of producing trimethylsulfoxonium bromide according to the present invention, the aforementioned minimum temperature is preferably lower than the aforementioned target reaction temperature but equal to or higher than 5.0°C below the aforementioned target reaction temperature

In the method of producing trimethylsulfoxonium bromide according to the present invention, while a reaction takes place between dimethyl sulfoxide and methyl bromide, the reaction vessel in which the reaction is carried out is preferably heated, and more preferably heating of the reaction vessel is carried out constantly.

In the method of producing trimethylsulfoxonium bromide according to the present invention, heating the reaction vessel so that its temperature can be maintained at from 0°C to 20°C above the aforementioned target reaction temperature is preferable. Furthermore, heating the aforementioned reaction vessel at a constant temperature is more preferable.

In the method of producing trimethylsulfoxonium bromide according to the present invention, the inclusion in the aforementioned reaction system of at least one compound selected from the group consisting of trimethyl orthoformate, triethyl orthoformate, tripropyl orthoformate, tetramethyl orthocarbonate, tetraethyl orthocarbonate, tetraisopropyl orthocarbonate, and tetrapropyl orthocarbonate in the reaction system is preferable.

In the method of producing trimethylsulfoxonium bromide according to the present invention, the moisture concentration in the aforementioned reaction system is preferably not greater than 400 ppm.

In the method of producing trimethylsulfoxonium bromide according to the present invention, the reaction of dimethyl sulfoxide and methyl bromide is preferably carried out under micropressure conditions.

Additionally, in the method of temperature control of a reaction system that produces trimethylsulfoxonium bromide according to the present invention, additional heating of the reaction system is preferable. Furthermore, heating the reaction system at a constant temperature is more preferable.

Embodiments of the present invention will be described in further detail hereinafter using practical examples. Of course, the present invention is not limited to the practical examples below, and it goes without saying that various modes are possible with regard to the details thereof. Furthermore, the present invention is not limited to the embodiments described above, and various modifications are possible within the scope described in the claims. Embodiments obtained by appropriately combining the technical means disclosed by the embodiments are also included in the technical scope of the present invention. In addition, all of the documents cited in this specification are hereby incorporated by reference.

### EXAMPLES

A 500 ml four-neck flask was fitted with a reaction solution temperature indicator, a methyl bromide introducing tube, a stirring apparatus with a vacuum seal, and a cooling apparatus with a Dewar type trap (for dry ice/acetone) connected to the top of a Dimroth type condenser (circulating coolant at -20°C). Under a nitrogen stream, 231 g of dimethyl sulfoxide and 3 g of trimethyl orthoformate were fed and heated. The tip of the cooling apparatus was sealed via a nitrogen stream during the reaction.

The temperature of the oil bath was held at from 67 to 68°C during the reaction. The instillation rate of methyl bromide was reduced or suspended when the reaction solution temperature fell below 63°C. Instillation resumed upon the temperature rising above 64°C, or alternatively, the reaction was continued through maintenance of the reaction solution temperature at from 62 to 65°C by accelerating the instillation rate while maintaining moderate methyl bromide reflux. In the aforementioned manner, 155 g of methyl bromide was added to the reaction solution from a hyper glass cylinder over 79 hours. The reaction solution was adequately mixed since the reaction solution forms slurry as the reaction proceeds.

The system was stirred for an additional 16 hours at the same temperature following the completion of methyl bromide addition. A yellow reaction solution was cooled to room temperature, after which precipitating crystals were filtered off. These crystals were washed with toluene and dried under vacuum at 55°C.
Yield 168.02 g (white crystals)
Yield rate: 63.2%

### INDUSTRIAL APPLICABILITY

The present invention can be utilized in the production of intermediates of active ingredients of antimicrobial agents for agriculture and horticulture.

## Claims

1. A method of producing trimethylsulfoxonium bromide by reacting dimethyl sulfoxide with methyl bromide, wherein
methyl bromide is added to dimethyl sulfoxide in a manner that satisfies (1) and (2) below:
(1) methyl bromide is added at an addition rate that complies with a predetermined feeding profile based on a reaction rate estimated from a target reaction temperature,
(2) a temperature in a reaction system of dimethyl sulfoxide and methyl bromide is detected and methyl bromide is added upon the detected temperature reaching a predetermined maximum temperature.

2. The method of producing trimethylsulfoxonium bromide according to claim 1, wherein the maximum temperature is higher than the target reaction temperature but is equal to or less than 5.0°C above the target reaction temperature.

3. The method of producing trimethylsulfoxonium bromide according to claims 1 and 2, wherein the addition rate of methyl bromide is reduced or addition is suspended upon the detected temperature falling below a predetermined minimum temperature.

4. The method of producing trimethylsulfoxonium bromide according to claim 3, wherein the minimum temperature is lower than the target reaction temperature but is equal to or higher than 5.0°C below the target reaction temperature.

5. The method of producing trimethylsulfoxonium bromide according to any one of claims 1 to 4, wherein a reaction vessel in which the reaction of dimethyl sulfoxide and methyl bromide is carried out is heated during the reaction.

6. The method of producing trimethylsulfoxonium bromide according to claim 5, wherein the reaction vessel is heated so that a temperature of the reaction vessel can be maintained at from 0°C to 20°C above the target reaction temperature.

7. The method of producing trimethylsulfoxonium bromide according to any one of claims 1 to 6, wherein at least one compound selected from the group consisting of trimethyl orthoformate, triethyl orthoformate, tripropyl orthoformate, tetramethyl orthocarbonate, tetraethyl orthocarbonate, tetraisopropyl orthocarbonate, and tetrapropyl orthocarbonate is contained in the reaction system.

8. The method of producing trimethylsulfoxonium bromide according to claim 7, wherein a moisture concentration in the reaction system is not greater than 400 ppm.

9. The method of producing trimethylsulfoxonium bromide according to any one of claims 1 to 8, wherein the reaction of dimethyl sulfoxide and methyl bromide is carried out at micropressure conditions.

10. A method of producing trimethylsulfoxonium bromide by reacting dimethyl sulfoxide with methyl bromide, wherein
methyl bromide is added to dimethyl sulfoxide in a manner that satisfies (1) and (2) below:
(1) a temperature in a reaction system is detected and methyl bromide is added upon the detected temperature reaching a predetermined maximum temperature,
(2) an addition rate of methyl bromide is reduced or addition is suspended upon the detected temperature falling below a predetermined minimum temperature.

11. A method of controlling a temperature in a reaction system that produces trimethylsulfoxonium bromide by reacting dimethyl sulfoxide with methyl bromide, wherein
the temperature in the reaction system is detected, methyl bromide is added upon the detected temperature reaching a predetermined maximum temperature, and an addition rate of methyl bromide is reduced or addition is suspended upon the detected temperature falling below a predetermined minimum temperature, thereby controlling the temperature in the reaction system.

12. A method of controlling the temperature in the reaction system producing the trimethylsulfoxonium bromide described in claim 11, wherein the reaction system is further heated.
